# EUROPEAN PATENT APPLICATION

(11) **EP 2 067 402 A1**
(43) Date of publication of application: **10.06.2009**
(21) Application number: 07023771.4
(22) Date of filing: 07.12.2007
(51) Int. Cl.: A01K 67/027, C12N 5/06, C12N 15/85, C12N 15/87

(54) **Transponson-mediated mutagenesis in spermatogonial stem cells**

(71) Applicant: Max Delbrück Centrum für Molekulare Medizin (MDC) Berlin-Buch;, 13125 Berlin (DE)
(72) Inventor: Izsvak, Zsuzsanna, 13125 Berlin (DE); Ivics, Zoltan, 13125 Berlin (DE); Grabundzija, Ivana, 10119 Berlin (DE); Mates, Lajos, 13125 Berlin (DE); Fröhlich, Janine, 13187 Berlin (DE); Hübner, Norbert, 13158 Berlin (DE)
(74) Representative: Vossius & Partner

(57) **Abstract**

The present invention relates to a method for generating mutations in spermatogonial stem cells comprising introducing at least one transposon into the spermatogonial stem cells. The invention further relates to cells obtainable by the method of the invention, a method for linking physiological data with genetic information as well as a transposon. Further, the invention relates to a method of generating non-human animals comprising the mutated spermatogonial stem cells as well as to a anon-human animals comprising the mutated spermatogonial stem cell of the invention. Furthermore, the invention relates to a method of saturating a genetic locus with mutations in a spermatogonial stem cell and finally, the invention relates to a spermatogonial stem cell library comprising mutated spermatogonial stem cells of the invention.

SEQUENCE LISTING

<110>  Max-Delbrück-Centrum für Molekulare Medizin (MDC) Berlin-Buch

<120>  Transposon-mediated mutagenesis in spermatogonial stem cells

<130>  N3355 EP

<160>  12

<170>  PatentIn version 3.3

<210>  1
<211>  340
<212>  PRT
<213>  artificial sequence

<220>
<221>  source
<223>  /note="Description of artificial sequence: consensus sequence
reconstructed from several fish species"

<400>  1

Met Gly Lys Ser Lys Glu Ile Ser Gln Asp Leu Arg Lys Lys Ile Val
1               5                   10                  15

Asp Leu His Lys Ser Gly Ser Ser Leu Gly Ala Ile Ser Lys Arg Leu
            20                  25                  30

Lys Val Pro Arg Ser Ser Val Gln Thr Ile Val Arg Lys Tyr Lys His
        35                  40                  45

His Gly Thr Thr Gln Pro Ser Tyr Arg Ser Gly Arg Arg Arg Val Leu
    50                  55                  60

Ser Pro Arg Asp Glu Arg Thr Leu Val Arg Lys Val Gln Ile Asn Pro
65                  70                  75                  80

Arg Thr Thr Ala Lys Asp Leu Val Lys Met Leu Glu Glu Thr Gly Thr
                85                  90                  95

Lys Val Ser Ile Ser Thr Val Lys Arg Val Leu Tyr Arg His Asn Leu
            100                 105                 110

Lys Gly Arg Ser Ala Arg Lys Lys Pro Leu Leu Gln Asn Arg His Lys
        115                 120                 125

Lys Ala Arg Leu Arg Phe Ala Thr Ala His Gly Asp Lys Asp Arg Thr
    130                 135                 140

Phe Trp Arg Asn Val Leu Trp Ser Asp Glu Thr Lys Ile Glu Leu Phe
145                 150                 155                 160

Gly His Asn Asp His Arg Tyr Val Trp Arg Lys Lys Gly Glu Ala Cys
                165                 170                 175

Lys Pro Lys Asn Thr Ile Pro Thr Val Lys His Gly Gly Gly Ser Ile
            180                 185                 190

Met Leu Trp Gly Cys Phe Ala Ala Gly Gly Thr Gly Ala Leu His Lys
        195                 200                 205

Ile Asp Gly Ile Met Arg Lys Glu Asn Tyr Val Asp Ile Leu Lys Gln
    210                 215                 220

His Leu Lys Thr Ser Val Arg Lys Leu Lys Leu Gly Arg Lys Trp Val
225                 230                 235                 240

Phe Gln Met Asp Asn Asp Pro Lys His Thr Ser Lys Val Val Ala Lys
                245                 250                 255

Trp Leu Lys Asp Asn Lys Val Lys Val Leu Glu Trp Pro Ser Gln Ser
            260                 265                 270

Pro Asp Leu Asn Pro Ile Glu Asn Leu Trp Ala Glu Leu Lys Lys Arg
        275                 280                 285

Val Arg Ala Arg Arg Pro Thr Asn Leu Thr Gln Leu His Gln Leu Cys
    290                 295                 300

Gln Glu Glu Trp Ala Lys Ile His Pro Thr Tyr Cys Gly Lys Leu Val
305                 310                 315                 320

Glu Gly Tyr Pro Lys Arg Leu Thr Gln Val Lys Gln Phe Lys Gly Asn
                325                 330                 335

Ala Thr Lys Tyr
            340

<210>  2
<211>  225
<212>  DNA
<213>  Tanichthys albonubes

<400>  2
cagttgaagt cggaagttta catacactta agttggagtc attaaaactc gtttttcaac      60

tacaccacaa atttcttgtt aacaaacaat agttttggca agtcagttag gacatctact     120

ttgtgcatga cacaagtcat ttttccaaca attgtttaca gacagattat ttcacttata     180

attcactgta tcacaattcc agtgggtcag aagtttacat acact                     225

<210>  3

<211>  226
<212>  DNA
<213>  Tanichthys albonubes

<400>  3
agtgtatgtt aacttctgac ccactgggaa tgtgatgaaa gaaataaaag ctgaaatgaa      60

tcattctctc tactattatt ctgatatttc acattcttaa aataaagtgg tgatcctaac     120

tgaccttaag acagggaatc tttactcgga ttaaatgtca ggaattgtga aaaagtgagt     180

ttaaatgtat ttggctaagg tgtatgtaaa cttccgactt caactg                    226


<210>  4
<211>  340
<212>  PRT
<213>  Rana pipiens

<400>  4

Met Pro Arg Pro Lys Glu Ile Gln Glu Gln Leu Arg Lys Lys Val Ile
1               5                   10                  15

Glu Ile Tyr Gln Ser Gly Lys Gly Tyr Lys Ala Ile Ser Lys Ala Leu
            20                  25                  30

Gly Ile Gln Arg Thr Thr Val Arg Ala Ile Ile His Lys Trp Arg Arg
        35                  40                  45

His Gly Thr Val Val Asn Leu Pro Arg Ser Gly Arg Pro Pro Lys Ile
    50                  55                  60

Thr Pro Arg Ala Gln Arg Arg Leu Ile Gln Glu Val Thr Lys Asp Pro
65                  70                  75                  80

Thr Thr Thr Ser Lys Glu Leu Gln Ala Ser Leu Ala Ser Val Lys Val
                85                  90                  95

Ser Val His Ala Ser Thr Ile Arg Lys Arg Leu Gly Lys Asn Gly Leu
            100                 105                 110

His Gly Arg Val Pro Arg Arg Lys Pro Leu Leu Ser Lys Lys Asn Ile
        115                 120                 125

Lys Ala Arg Leu Asn Phe Ser Thr Thr His Leu Asp Asp Pro Gln Asp
    130                 135                 140

Phe Trp Asp Asn Ile Leu Trp Thr Asp Glu Thr Lys Val Glu Leu Phe
145                 150                 155                 160

Gly Arg Cys Val Ser Lys Tyr Ile Trp Arg Arg Arg Asn Thr Ala Phe
                165                 170                 175

His Lys Lys Asn Ile Ile Pro Thr Val Lys Tyr Gly Gly Gly Ser Val
            180                 185                 190

Met Val Trp Gly Cys Phe Ala Ala Ser Gly Pro Gly Arg Leu Ala Val
        195                 200                 205

Ile Lys Gly Thr Met Asn Ser Ala Val Tyr Gln Glu Ile Leu Lys Glu
    210                 215                 220

Asn Val Arg Pro Ser Val Arg Val Leu Lys Leu Lys Arg Thr Trp Val
225                 230                 235                 240

Leu Gln Gln Asp Asn Asp Pro Lys His Thr Ser Lys Ser Thr Thr Glu
                245                 250                 255

Trp Leu Lys Lys Asn Lys Met Lys Thr Leu Glu Trp Pro Ser Gln Ser
            260                 265                 270

Pro Asp Leu Asn Pro Ile Glu Met Leu Trp Tyr Asp Leu Lys Lys Ala
        275                 280                 285

Val His Ala Arg Lys Pro Ser Asn Val Thr Glu Leu Gly Gln Phe Cys
    290                 295                 300

Lys Asp Glu Trp Ala Lys Ile Pro Pro Gly Arg Cys Lys Ser Leu Ile
305                 310                 315                 320

Ala Arg Tyr Arg Lys Arg Leu Val Ala Val Val Ala Ala Lys Gly Gly
                325                 330                 335

Pro Thr Ser Tyr
            340

<210>  5
<211>  214
<212>  DNA
<213>  Rana pipiens

<400>  5
cagtggtgtg aaaaagtgtt tgcccccttc ctcatttcct gttcctttgc atgtttgtca      60

cacttaagtg tttcggaaca tcaaaccaat ttaaacaata gtcaaggaca acacaagtaa     120

acacaaaatg caatttgtaa atgaaggtgt ttattattaa aggtgaaaaa aaatccaaac     180

catcatggcc ctgtgtgaaa aagtgattgc cccc                                 214

<210>  6
<211>  214
<212>  DNA
<213>  Rana pipiens

<400>  6
gggggcaatc actttttcac acagggccat gatggtttgg attttttttc acctttaata      60

ataaacacct tcatttacaa attgcatttt gtgtttactt gtgttgtcct tgactattgt     120

ttaaattggt ttgatgttcc gaaacactta agtgtgacaa acatgcaaag gaacaggaaa     180

tgaggaaggg ggcaaacact ttttcacacc actg                                  214


<210>  7
<211>  649
<212>  PRT
<213>  Oryzias latipes

<400>  7

Met Glu Glu Val Cys Asp Ser Ser Ala Ala Ala Ser Ser Thr Val Gln
1               5                   10                  15

Asn Gln Pro Gln Asp Gln Glu His Pro Trp Pro Tyr Leu Arg Glu Phe
            20                  25                  30

Phe Ser Leu Ser Gly Val Asn Lys Asp Ser Phe Lys Met Lys Cys Val
        35                  40                  45

Leu Cys Leu Pro Leu Asn Lys Glu Ile Ser Ala Phe Lys Ser Ser Pro
    50                  55                  60

Ser Asn Leu Arg Lys His Ile Glu Arg Met His Pro Asn Tyr Leu Lys
65                  70                  75                  80

Asn Tyr Ser Lys Leu Thr Ala Gln Lys Arg Lys Ile Gly Thr Ser Thr
                85                  90                  95

His Ala Ser Ser Ser Lys Gln Leu Lys Val Asp Ser Val Phe Pro Val
            100                 105                 110

Lys His Val Ser Pro Val Thr Val Asn Lys Ala Ile Leu Arg Tyr Ile
        115                 120                 125

Ile Gln Gly Leu His Pro Phe Ser Thr Val Asp Leu Pro Ser Phe Lys
    130                 135                 140

Glu Leu Ile Ser Thr Leu Gln Pro Gly Ile Ser Val Ile Thr Arg Pro
145                 150                 155                 160

Thr Leu Arg Ser Lys Ile Ala Glu Ala Ala Leu Ile Met Lys Gln Lys
                165                 170                 175

Val Thr Ala Ala Met Ser Glu Val Glu Trp Ile Ala Thr Thr Thr Asp
            180                 185                 190

Cys Trp Thr Ala Arg Arg Lys Ser Phe Ile Gly Val Thr Ala His Trp
        195                 200                 205

Ile Asn Pro Gly Ser Leu Glu Arg His Ser Ala Ala Leu Ala Cys Lys
    210                 215                 220

Arg Leu Met Gly Ser His Thr Phe Glu Val Leu Ala Ser Ala Met Asn
225                 230                 235                 240

Asp Ile His Ser Glu Tyr Glu Ile Arg Asp Lys Val Val Cys Thr Thr
                245                 250                 255

Thr Asp Ser Gly Ser Asn Phe Met Lys Ala Phe Arg Val Phe Gly Val
            260                 265                 270

Glu Asn Asn Asp Ile Glu Thr Glu Ala Arg Arg Cys Glu Ser Asp Asp
        275                 280                 285

Thr Asp Ser Glu Gly Cys Gly Glu Gly Ser Asp Gly Val Glu Phe Gln
    290                 295                 300

Asp Ala Ser Arg Val Leu Asp Gln Asp Asp Gly Phe Glu Phe Gln Leu
305                 310                 315                 320

Pro Lys His Gln Lys Cys Ala Cys His Leu Leu Asn Leu Val Ser Ser
                325                 330                 335

Val Asp Ala Gln Lys Ala Leu Ser Asn Glu His Tyr Lys Lys Leu Tyr
            340                 345                 350

Arg Ser Val Phe Gly Lys Cys Gln Ala Leu Trp Asn Lys Ser Ser Arg
        355                 360                 365

Ser Ala Leu Ala Ala Glu Ala Val Glu Ser Glu Ser Arg Leu Gln Leu
    370                 375                 380

Leu Arg Pro Asn Gln Thr Arg Trp Asn Ser Thr Phe Met Ala Val Asp
385                 390                 395                 400

Arg Ile Leu Gln Ile Cys Lys Glu Ala Gly Glu Gly Ala Leu Arg Asn
                405                 410                 415

Ile Cys Thr Ser Leu Glu Val Pro Met Phe Asn Pro Ala Glu Met Leu
            420                 425                 430

Phe Leu Thr Glu Trp Ala Asn Thr Met Arg Pro Val Ala Lys Val Leu
        435                 440                 445

Asp Ile Leu Gln Ala Glu Thr Asn Thr Gln Leu Gly Trp Leu Leu Pro
    450                 455                 460

Ser Val His Gln Leu Ser Leu Lys Leu Gln Arg Leu His His Ser Leu
465                 470                 475                 480

Arg Tyr Cys Asp Pro Leu Val Asp Ala Leu Gln Gln Gly Ile Gln Thr
                485                 490                 495

Arg Phe Lys His Met Phe Glu Asp Pro Glu Ile Ile Ala Ala Ala Ile
            500                 505                 510

Leu Leu Pro Lys Phe Arg Thr Ser Trp Thr Asn Asp Glu Thr Ile Ile
        515                 520                 525

Lys Arg Gly Met Asp Tyr Ile Arg Val His Leu Glu Pro Leu Asp His
    530                 535                 540

Lys Lys Glu Leu Ala Asn Ser Ser Ser Asp Asp Glu Asp Phe Phe Ala
545                 550                 555                 560

Ser Leu Lys Pro Thr Thr His Glu Ala Ser Lys Glu Leu Asp Gly Tyr
                565                 570                 575

Leu Ala Cys Val Ser Asp Thr Arg Glu Ser Leu Leu Thr Phe Pro Ala
            580                 585                 590

Ile Cys Ser Leu Ser Ile Lys Thr Asn Thr Pro Leu Pro Ala Ser Ala
        595                 600                 605

Ala Cys Glu Arg Leu Phe Ser Thr Ala Gly Leu Leu Phe Ser Pro Lys
    610                 615                 620

Arg Ala Arg Leu Asp Thr Asn Asn Phe Glu Asn Gln Leu Leu Leu Lys
625                 630                 635                 640

Leu Asn Leu Arg Phe Tyr Asn Phe Glu
                645

<210>  8
<211>  150
<212>  DNA
<213>  Oryzias latipes

<400>  8
cagaggtgta aaaagtactc aaaaatttta ctcaagtgaa agtacaagta cttagggaaa      60

attttactca attaaaagta aaagtatctg gctagaatct tacttgagta aaagtaaaaa     120

agtactccat taaaattgta cttgagtatt                                    150

<210>  9
<211>  200
<212>  DNA
<213>  Oryzias latipes

<400>  9
aagtgatctc caaaaaataa gtactttttg actgtaaata aaattgtaag gagtaaaaag      60

tacttttttt tctaaaaaaa tgtaattaag taaaagtaaa agtattgatt tttaattgta     120

ctcaagtaaa gtaaaaatcc ccaaaaataa tacttaagta cagtaatcaa gtaaaattac     180

tcaagtactt tacacctctg                                                200

<210>  10
<211>  594
<212>  PRT
<213>  Trichoplusia ni

<400>  10

Met Gly Ser Ser Leu Asp Asp Glu His Ile Leu Ser Ala Leu Leu Gln
1               5                   10                  15

Ser Asp Asp Glu Leu Val Gly Glu Asp Ser Asp Ser Glu Ile Ser Asp
            20                  25                  30

His Val Ser Glu Asp Asp Val Gln Ser Asp Thr Glu Glu Ala Phe Ile
        35                  40                  45

Asp Glu Val His Glu Val Gln Pro Thr Ser Ser Gly Ser Glu Ile Leu
    50                  55                  60

Asp Glu Gln Asn Val Ile Glu Gln Pro Gly Ser Ser Leu Ala Ser Asn
65                  70                  75                  80

Arg Ile Leu Thr Leu Pro Gln Arg Thr Ile Arg Gly Lys Asn Lys His
                85                  90                  95

Cys Trp Ser Thr Ser Lys Ser Thr Arg Arg Ser Arg Val Ser Ala Leu
            100                 105                 110

Asn Ile Val Arg Ser Gln Arg Gly Pro Thr Arg Met Cys Arg Asn Ile
        115                 120                 125

Tyr Asp Pro Leu Leu Cys Phe Lys Leu Phe Phe Thr Asp Glu Ile Ile
    130                 135                 140

Ser Glu Ile Val Lys Trp Thr Asn Ala Glu Ile Ser Leu Lys Arg Arg
145                 150                 155                 160

Glu Ser Met Thr Gly Ala Thr Phe Arg Asp Thr Asn Glu Asp Glu Ile
                165                 170                 175

Tyr Ala Phe Phe Gly Ile Leu Val Met Thr Ala Val Arg Lys Asp Asn
            180                 185                 190

His Met Ser Thr Asp Asp Leu Phe Asp Arg Ser Leu Ser Met Val Tyr
        195                 200                 205

Val Ser Val Met Ser Arg Asp Arg Phe Asp Phe Leu Ile Arg Cys Leu
    210                 215                 220

Arg Met Asp Asp Lys Ser Ile Arg Pro Thr Leu Arg Glu Asn Asp Val
225                 230                 235                 240

Phe Thr Pro Val Arg Lys Ile Trp Asp Leu Phe Ile His Gln Cys Ile
                245                 250                 255

Gln Asn Tyr Thr Pro Gly Ala His Leu Thr Ile Asp Glu Gln Leu Leu
            260                 265                 270

Gly Phe Arg Gly Arg Cys Pro Phe Arg Met Tyr Ile Pro Asn Lys Pro
        275                 280                 285

Ser Lys Tyr Gly Ile Lys Ile Leu Met Met Cys Asp Ser Gly Thr Lys
    290                 295                 300

Tyr Met Ile Asn Gly Met Pro Tyr Leu Gly Arg Gly Thr Gln Thr Asn
305                 310                 315                 320

Gly Val Pro Leu Gly Glu Tyr Tyr Val Lys Glu Leu Ser Lys Pro Val
                325                 330                 335

His Gly Ser Cys Arg Asn Ile Thr Cys Asp Asn Trp Phe Thr Ser Ile
            340                 345                 350

Pro Leu Ala Lys Asn Leu Leu Gln Glu Pro Tyr Lys Leu Thr Ile Val
        355                 360                 365

Gly Thr Val Arg Ser Asn Lys Arg Glu Ile Pro Glu Val Leu Lys Asn
    370                 375                 380

Ser Arg Ser Arg Pro Val Gly Thr Ser Met Phe Cys Phe Asp Gly Pro
385                 390                 395                 400

Leu Thr Leu Val Ser Tyr Lys Pro Lys Pro Ala Lys Met Val Tyr Leu
                405                 410                 415

Leu Ser Ser Cys Asp Glu Asp Ala Ser Ile Asn Glu Ser Thr Gly Lys
            420                 425                 430

Pro Gln Met Val Met Tyr Tyr Asn Gln Thr Lys Gly Gly Val Asp Thr
        435                 440                 445

Leu Asp Gln Met Cys Ser Val Met Thr Cys Ser Arg Lys Thr Asn Arg
    450                 455                 460

Trp Pro Met Ala Leu Leu Tyr Gly Met Ile Asn Ile Ala Cys Ile Asn
465                 470                 475                 480

Ser Phe Ile Ile Tyr Ser His Asn Val Ser Ser Lys Gly Glu Lys Val
                485                 490                 495

Gln Ser Arg Lys Lys Phe Met Arg Asn Leu Tyr Met Ser Leu Thr Ser
            500                 505                 510

Ser Phe Met Arg Lys Arg Leu Glu Ala Pro Thr Leu Lys Arg Tyr Leu
        515                 520                 525

Arg Asp Asn Ile Ser Asn Ile Leu Pro Asn Glu Val Pro Gly Thr Ser
    530                 535                 540

Asp Asp Ser Thr Glu Glu Pro Val Met Lys Lys Arg Thr Tyr Cys Thr
545                 550                 555                 560

Tyr Cys Pro Ser Lys Ile Arg Arg Lys Ala Asn Ala Ser Cys Lys Lys
                565                 570                 575

Cys Lys Lys Val Ile Cys Arg Glu His Asn Ile Asp Met Cys Gln Ser
            580                 585                 590

Cys Phe

<210> 11
<211> 306
<212> DNA
<213> Trichoplusia ni

<400> 11
ccctagaaag atagtctgcg taaaattgac gcatgcattc ttgaaatatt gctctctctt 60

tctaaatagc gcgaatccgt cgctgtgcat ttaggacatc tcagtcgccg cttggagctc 120

ccgtgaggcg tgcttgtcaa tgcggtaagt gtcactgatt ttgaactata acgaccgcgt 180

gagtcaaaat gacgcatgat tatcttttac gtgactttta agatttaact catacgataa 240

ttatattgtt atttcatgtt ctacttacgt gataacttat tatatatata ttttcttgtt 300

atagat                                                              306

<210>  12
<211>  237
<212>  DNA
<213>  Trichoplusia ni

<400>  12
aaaagttttg ttactttata gaagaaattt tgagtttttg ttttttttta ataaataaat      60

aaacataaat aaattgtttg ttgaatttat tattagtatg taagtgtaaa tataataaaa     120

cttaatatct attcaaatta ataaataaac ctcgatatac agaccgataa aacacatgcg     180

tcaattttac gcatgattat ctttaacgta cgtcacaata tgattatctt tctaggg        237

## Description

The present invention relates to a method for generating mutations in spermatogonial stem cells comprising introducing at least one transposon into the spermatogonial stem cells. The invention further relates to cells obtainable by the method of the invention, a method for linking physiological data with genetic information and also relates to a transposon. Further, the invention relates to a method of generating non-human animals comprising the mutated spermatogonial stem cells as well as to a non-human animals comprising the mutated spermatogonial stem cell of the invention Furthermore, the invention relates to a method of saturating a genetic locus with mutations in a spermatogonial stem cell and finally, the invention relates to a spermatogonial stem cell library comprising mutated spermatogonial stem cells of the invention.

In the specification a number of documents is cited. The disclosure content of these documents including manufacturers' manuals is herewith incorporated by reference.

Given the need to annotate the human genome with function, linking laboratory animals into this process is a necessary requirement for accelerating improvements in health care. For example, extensive phenotyping and detailed analysis of inbred animals strains has resulted in the localisation of hundreds of loci involved in complex diseases. These "quantitative trait loci" (QTLs) demonstrate genetic linkage to many disease traits which are shared between laboratory animals, such as rats, and humans. Examples for such diseases include hypertension, neuronal regeneration, ischemic cerebro- and cardiovascular diseases and diabetes (Jacob, H.J. and A.E. Kwitek, 2002, "Rat genetics: attaching physiology and pharmacology to the Genome" Nat Rev Genet 3(1):33; Hubner, N. et al., 2005, "Integrated transcriptional profiling and linkage analysis for identification of genes underlying disease" Nat Genet 37(3):243).

In mice, embryonic stem (ES) cell based knockout technology is very efficient for single gene targeting and can also be combined with the usage of random mutagens such as chemical mutagenic agents, viruses or transposons for the large scale generation of ES cell libraries carrying different molecularly marked knockout alleles. These ES cell clones can efficiently be used for the production of knockout mice. However, while this method is applicable for mice it cannot be applied to other laboratory animals, such as rat. Furthermore, no similar or equivalent techniques to the mouse ES cell technique have yet been developed that would be applicable to a variety of animal models and not limited to one animal species like the ES cell technique in mice. For example, due to the above-mentioned technical limitation very few rat knockout strains are currently existing worldwide. This may at least partially be the result of the practicability of random mutagenesis in animals which has proven to be questionable for several reasons. For example, the requirement of a large number of offspring, the time for rearing offspring, the costs of establishing and maintaining large-scale animal facilities are some of the factors to be considered when generating transgenic animals using random mutagenesis in animals. Thus, there exists a need for more advantageous methods of targeted mutagenesis that can be applied in a variety of animal models and are more practicable.

The technical problem underlying the present invention is the provision of an alternative method that allows the successful generation of a variety of knockout or transgenic animal models.

The solution to this technical problem is achieved by the embodiments characterised in the claims.

Accordingly, the present invention relates to a method for generating mutations in spermatogonial stem cells comprising introducing at least one transposon into the spermatogonial stem cells.

The term "mutation" in accordance with the present invention refers to changes in the base pair sequence of the genetic material of an organism. Mutations include for example point mutations, duplications, inversions, insertions or deletions. Point mutations refer to an exchange of a single nucleotide for another nucleotide and, when they occur within a protein coding region of a gene may be classified into three kinds, namely silent mutations, which code for the same amino acid, missense mutations, which code for a different amino acid or nonsense mutations, which result in truncation of the protein. Duplications refer to the amplification of DNA segment comprising at least one single nucleotide. Such an amplification may, for example, affect a complete genome leading to polyploid cells. Inversions refer to the change in orientation of a DNA segment comprising at least two consecutive nucleotides. Insertions refer to the addition of one or more extra nucleotides, entire genes or even chromosomal regions into the DNA. Where an insertions occurrs in the coding region of a gene it may alter the splicing of the mRNA (splice site mutation), or cause a shift in the reading frame (frameshift), both of which can significantly alter the gene product. Furthermore, insertions can lead to a disruption of the reading frame resulting in abolished gene expression. In addition, insertion of genes or chromosomal regions may result in the expression of additional, foreign, gene products or the increase of gene expression of native gene expression products. Deletions refer to the removal of one or more extra nucleotides, entire genes or chromosomal regions from the DNA. Like insertions, these mutations can alter the reading frame of the gene. Consequently, the introduction of a transposon in accordance with the method of the invention can be regarded as an insertion, whereas the excision can be considered a deletion. Some transposons are not entirely excised and nucleotide insertions are left behind at the site of the preceding transposon integration. Further, point mutations, duplications or inversions may be simultaneously introduced as part of a transgene comprised in said transposon. Thus, simultaneously several different mutations may be generated in spermatogonial stem cells.

The term "spermatogonial stem cells" in accordance with the present invention refers to adult stem cells isolated from the testis. Spermatogonial stem cells are incapable of fertilising an egg cell but can give rise to cells that develop into sperm and that produce viable offspring. Isolated spermatogonial stem cells can be cultured for a prolonged time period without losing their properties and can efficiently repopulate the testes of suitable recipient male animals (Oatley JM and Brinster RL, 2006, "Spermatogonial stem cells". Methods Enzymol. 419:259).

The term "transposon" refers to segments of DNA that can move (transpose) within the genome. A transposon in accordance with the present invention may or may not encode the enzyme transposase, necessary to catalyze its relocation and/or duplication in the genome. Where a transposon does not encode for its transposase enzyme, expression of said enzyme in trans may be required when carrying out the method of the invention in cells not expressing the relevant transposase itself. Furthermore, the transposon must contain sequences that are required for its mobilization, namely the terminal inverted repeats containing the binding sites for the transposase. The transposon may be derived from a bacterial or a eukaryotic transposon. Further, the transposon may be derived from a class I or class II transposon. Class II or DNA-mediated transposable elements are preferred for gene transfer applications, because transposition of these elements does not involve a reverse transcription step (involved in transposition of Class I or retroelements) which can introduce undesired mutations into transgenes (Miller, A.D., 1997, "Development and applications of retroviral vectors". Retroviruses:843 (Cold Spring Harbor Laboratory Press, New York,); Verma, I.M. and Somia, N., 1997, "Gene therapy? promises, problems and prospects". Nature 389:239). Apart from naturally occurring transposons, also modified transposon systems such as those disclosed in EP1594973, EP1594971 or EP1594972 may be employed in the method of the invention. Preferably, the transposons used for the method of the invention possess highly elevated transpositional activity.

Transposons are invaluable genomic tools in invertebrates and have gained importance for vertebrate genomics as well. Transposons can be harnessed as vehicles for introducing mutations into genomes. Genes may, for example, be inactivated by transposon insertion. Such genes are then "tagged" by the transposable element, which can be used for subsequent cloning of the mutated allele. In addition to gene inactivation, a transposon may also introduce a transgene of interest into the genome. The transposon may further carry the regulatory elements necessary for the expression of said transgene, thus allowing for successful expression of the gene.

Accordingly, a powerful approach of introducing mutations into the genome is insertional mutagenesis mediated by transposons. Discrete pieces of foreign DNA can be harnessed to disrupt host gene function by creating random insertions in the genome. As opposed to chemical mutagenesis, inserting DNA fragments into genes simultaneously provide a molecular tag, which can be used to rapidly identify the mutated allele. Viral and non-viral technologies have been devised to facilitate the penetration of transgenes through biological membranes.
When transposons are used in insertional mutagenesis screens, transposon vectors often comprise four major classes of constructs to identify the mutated genes rapidly. These contain a reporter gene, which should be expressed depending on the genetic context of the integration. In *enhancer traps,* the expression of the reporter requires the presence of a genomic cis-regulator to act on an attenuated promoter within the integrated construct. *Promoter traps* contain no promoter at all. These vectors are only expressed if they land in-frame in an exon or close downstream to a promoter of an expressed gene. In *polyA traps,* the marker gene lacks a polyA signal, but contains a splice donor (SD) site. Thus, when integrating into an intron, a fusion transcript can be synthesized comprising the marker and the downstream exons of the trapped gene. *Gene traps* (or exon traps) also lack promoters, but are equipped with a splice acceptor (SA) preceding the marker gene. Reporter activation occurs if the vector is integrated into an expressed gene, and splicing between the reporter and an upstream exon takes place. The gene trap and polyA trap cassettes can be combined. In that case, the marker of the polyA trap part is amended with a promoter so that the vector can also trap downstream exons, and both upstream and downstream fusion transcripts of the trapped gene can be obtained. The above constructs also offer the possibility to visualize spatial and temporal expression patterns of the mutated genes by using, e.g., *LacZ* or fluorescent proteins as a marker gene.

A milestone in the use of transposons in vertebrate genomics was the creation of the *Sleeping Beauty* (SB) element (Ivics, Z. et al., 1997: "Molecular reconstruction of Sleeping Beauty, a Tcl-like transposon from fish, and its transposition in human cells". Cell 91(4):501). Sleeping Beauty transposition is efficient in cells of different vertebrate classes (Izsvak, Z. et al., 2000: "Sleeping Beauty, a wide host-range transposon vector for genetic transformation in vertebrates". J Mol Biol. 302(1):93; Lu, B. et al., 2007: "Generation of rat mutants using a coat color-tagged Sleeping Beauty transposon system". Mamm Genome 18(5):338; Luo, G. et al., 1998: "Chromosomal transposition of a Tc1/mariner-like element in mouse embryonic stem cells". Proc Natl Acad Sci U S A 95(18):10769; Horie, K. et al., 2003: "Characterization of Sleeping Beauty transposition and its application to genetic screening in mice". Mol Cell Biol 23(24):9189). First mutagenesis screens have established that Sleeping Beauty can generate a high number of random mutations in mouse and rat germinal cells *in vivo* (Kitada, K. et al., 2007: "Transposon-tagged mutagenesis in the rat". Nat Methods 4(2):131; Lu, B. et al., 2007: "Generation of rat mutants using a coat color-tagged Sleeping Beauty transposon system". Mamm Genome 18(5):338).

The use of gene-trap constructs for insertional mutagenesis in tissue culture, where trapped events can easily be selected for, is advantageous over the random mutagenesis in animals. Cells selected for a particular gene trap event can be used for the generation of animal models lacking this specific genetic function. However, while for example embryonic stem (ES) technology is readily available for mice, this technology cannot be applied to other animals such as rat. As an alternative approach, in accordance with the invention, the use of spermatogonial stem cells allows for gene-specific inactivation and manipulation of the germline of a wide variety of non-human animals, in particular in rat, where such a technology was previously lacking. Thus, the present invention provides a method based on the combination of transposon-mediated insertional mutagenesis with a tissue culture system, i.e. spermatogonial stem cells, that allows for the convenient generation of *in vitro* spermatogonial stem cell libraries carrying a large number of different insertion events. Furthermore, compared to classical nuclear transfer technologies and *in vivo* mutagenesis this method is less cost- and labour intensive and allows for the selection of a the appropriate insertion before establishing the corresponding animal models. In addition, using these cells or libraries, a wider variety of animal models can be established.

In a preferred embodiment additional regulatory elements are introduced into the spermatogonial stem cells. For example, by combining transposon elements with the Cre-recombinase/loxP system, chromosomal deletions and/or inversions can be generated. Further elements are, for example, the Flp-recombinase/frt system that can be applied for Recombinase Mediated Cassette Exchange (RMCE) in order to knock-in alleles into a known chromosomal location, or the φC31 integrase-attP system that can be employed for site-specific integration.

The present invention also relates to a mutated spermatogonial stem cell, obtainable by the method of the invention. The mutated spermatogonial stem cell that is obtainable or obtained by the method of the invention may be used to repopulate the testes of a suitable recipient male animal or, alternatively, may be frozen for storage. Cells can be kept frozen for at least one year and recovered back to culture, without any obvious morphological change. Recovered cells may also be used to repopulate the testes of recipient males. For the repopulation of testes, the spermatogonial stem cells are transplanted into the testes of sterilized recipient male animals (Kubota H and Brinster RL., 2006, "Technology insight: In vitro culture of spermatogonial stem cells and their potential therapeutic uses." Nat Clin Pract Endocrinol Metab. 2(2):99). For example, approximately 0.5×10⁶ cultured spermatogonial stem cells are required for successful transplantation in rats. About 50 - 80% of the offspring will generally originate from the transferred spermatogonial stem cells. Suitable cell numbers used for transplantation will vary dependent on the species. However, the person skilled in the art knows how to determine appropriate cell numbers.

In a preferred embodiment of the method or the mutated spermatogonial stem cell of the present invention, the at least one transposon is a eukaryotic transposon.

In a further preferred embodiment of the method or the mutated spermatogonial stem cell of the present invention, the transposon is the Sleeping Beauty transposon, the Frog Prince transposon, the piggyBac transposon or the Tol2 transposon.

A number of transposon systems that are able to also transpose in vertebrates have recently became available, such as Sleeping Beauty, piggyBac, Tol2 or Frog Prince. Sleeping Beauty (SB) and Frog Prince (FP) are Tc1 transposons, whereas piggyBac (PB) was the founder of the PB transposon family and Tol2 is a hAT transposon family member. Both the Sleeping Beauty and the Frog Prince transposon are found in vertebrates as inactive copies, from which active transposon systems have been engineered. The Tol2 transposon has also been found in vertebrates as an active transposon. The piggyBac transposon was originally found as an active transposon in insects but was subsequently shown to have high levels of activity also in vertebrates (Ding S et al., 2005, "Efficient transposition of the piggyBac (PB) transposon in mammalian cells and mice." Cell 122(3):473). Each of these elements has their own advantages, for example, Sleeping Beauty is particularly useful in region-specific mutagenesis, whereas Tol2 has the highest tendency to integrate into expressed genes. Hyperactive systems are available for Sleeping Beauty and piggyBac. Most importantly, these transposons have distinct target site preferences, and can therefore mutagenise overlapping, but distinct sets of genes. Therefore, to achieve the best possible coverage of genes, the use of more than one element is particularly preferred.
The Sleeping Beauty transposon is described, for example, in lzsvak, Z. et al., 2000, "Sleeping Beauty, a wide host-range transposon vector for genetic transformation in vertebrates". J Mol Biol 302(1):93. The Sleeping Beauty transposon recognition sites have the sequence of SEQ ID NOs: 2 and 3; the Sleeping Beauty transposase has the sequence of SEQ ID NO: 1. The Frog Prince transposon is described in German patent application 102 24 242.9. Its transposon recognition sites have the sequence of SEQ ID NOs: 5 and 6; the Frog Prince transposase has the sequence of SEQ ID NO: 4. The piggyBac transposon was described, for example, in Ding S et al., 2005, "Efficient transposition of the piggyBac (PB) transposon in mammalian cells and mice." Cell 122(3):473. Its transposon recognition sites have the sequence of SEQ ID NOs: 11 and 12; the piggyBac transposase has the sequence of SEQ ID NO: 10. The Tol2 transposon has been described, for example, in Balciunas D et al., 2006, "Harnessing a high cargo-capacity transposon for genetic applications in vertebrates." PLoS Genet. 2(11):e169. Its transposon recognition sites have the sequence of SEQ ID NOs: 8 and 9; the Tol2 transposase has the sequence of SEQ ID NO: 7.

In another preferred embodiment of the method or the mutated spermatogonial stem cell of the present invention, the transposon comprises additional nucleotide sequences selected from the group consisting of nucleotide sequences encoding a selectable marker, a transgene or an siRNA, shRNA or a ribozyme.

The term "nucleotide sequence" in accordance with the present invention include DNA, such as cDNA or genomic DNA, and synthetic or semi-synthetic derivatives of DNA and RNA.

The term "selectable marker" in accordance with the present invention refers to a protein that enables the separation of cells expressing said marker from cells devoid of the marker. For example, the selectable marker may be a fluorescent marker. Expression of the marker by cells having successfully integrated the transposon allows the isolation of these cells using methods such as, for example, FACS (fluorescent activated cell sorting). Alternatively, expression of a selectable marker may confer an advantageous property to the cell that allows survival of only those cells carrying the gene. For example, the marker protein may allow for the selection of the cell by conferring an antibiotic resistance to the cell. Consequently, when cells are cultured in medium containing said antibiotic, only cell clones expressing the marker protein that mediates antibiotic resistance are capable of propagating. For example, the marker protein may confer resistance to antibiotics such as ampicillin, kanamycin, chloramphenicol, tetracycline, hygromycin, neomycin or methotrexate. Further examples of antibiotics are penicillins: ampicillin HCl, ampicillin Na, amoxycillin Na, carbenicillin disodium, penicillin G, cephalosporins, cefotaxim Na, cefalexin HCl, vancomycin, cycloserine. Other examples include bacteriostatic inhibitors such as: chloramphenicol, erythromycin, lincomycin, spectinomycin sulfate, clindamycin HCl, chlortetracycline HCl. Additional examples are marker proteins that allow selection with bactericidal inhibitors such as those affecting protein synthesis irreversibly causing cell death, for example aminoglycosides such as gentamycin, hygromycin B, kanamycin, neomycin, streptomycin, G418, tobramycin. aminoglycosides can be inactivated by enzymes such as NPT II which phosphorylates 3'-OH present on kanamycin, thus inactivating this antibiotic. Some aminoglycoside modifying enzymes acetylate the compounds and block their entry in to the cell. Marker proteins that allow selection with nucleic acid metabolism inhibitors like rifampicin, mitomycin C, nalidixic acid, doxorubicin HCl, 5-flurouracil, 6-mercaptopurine, antimetabolites, miconazole, trimethoprim, methotrexate, metronidazole, sulfametoxazole are also examples for selectable markers.

A "transgene" according to the present invention is a foreign gene of interest. Alternatively, said transgene is a gene homologous to the cell into which it was introduced or into a precursor of which it was introduced but occurs at a different chromosomal position as compared to the corresponding endogenous gene. The nucleotide sequences encoding the transgene inserted in the transposon can e.g. be synthesized by standard methods, or isolated from natural sources. Ligation of the coding sequences to transcriptional regulatory elements and/or to other amino acid encoding sequences can be carried out using established methods. Transcriptional regulatory elements (parts of an expression cassette) ensuring expression in eukaryotic cells are well known to those skilled in the art. These elements comprise regulatory sequences ensuring the initiation of the transcription (e. g. translation initiation codon, promoters, enhancers, and/or insulators), internal ribosomal entry sites (IRES) (Owens Proc. 2001 Natl. Acad. Sci. USA, 98:1471) and optionally poly-A signals ensuring termination of transcription and stabilization of the transcript. Additional regulatory elements may include transcriptional as well as translational enhancers, and/or naturally-associated or heterologous promoter regions. Preferably, the transgene is operatively linked to such expression control sequences allowing expression in eukaryotic cells. Nucleotide sequences encoding secretion signals may further be comprised as additional regulatory elements. Such sequences are well known to the person skilled in the art. Furthermore, leader sequences capable of directing the expressed protein product of the transgene to a cellular compartment may be added to the coding sequence of the transgene. Such leader sequences are well known in the art.

Possible examples for regulatory elements ensuring the initiation of transcription comprise the cytomegalovirus (CMV) promoter, SV40-promoter, RSV-promoter (Rous sarcome virus), the lacZ promoter, the gai10 promoter, human elongation factor 1a-promoter, CMV enhancer, CaM-kinase promoter, the Autographa californica multiple nuclear polyhedrosis virus (AcMNPV) polyhedral promoter or the SV40-enhancer. Examples for further regulatory elements in prokaryotes and eukaryotic cells comprise transcription termination signals, such as SV40-poly-A site or the tk-poly-A site or the SV40, lacZ and AcMNPV polyhedral polyadenylation signals, downstream of the polynucleotide. Moreover, elements such as origin of replication and regulators (as part of an inducible promoter) may also be included. Additional elements might include enhancers, Kozak sequences and intervening sequences flanked by donor and acceptor sites for RNA splicing. Preferably, the regulatory elements are the native regulatory elements of the gene of interest.

The term "siRNA" in accordance to the present invention refers to small interfering RNA, also known as short interfering RNA or silencing RNA. siRNAs are a class of 18 to 30, preferably 20 to 25, most preferred 21 to 23 or 21 nucleotide-long double-stranded RNA molecules that play a variety of roles in biology. Most notably, siRNA is involved in the RNA interference (RNAi) pathway where the siRNA interferes with the expression of a specific gene. In addition to their role in the RNAi pathway, siRNAs also act in RNAi-related pathways, e.g. as an antiviral mechanism or in shaping the chromatin structure of a genome. siRNAs have a well defined structure: a short double-strand of RNA (dsRNA), advantageously with one RNA strand having anoverhang. Each strand has a 5' phosphate group and a 3' hydroxyl (-OH) group. This structure is the result of processing by dicer, an enzyme that converts either long dsRNAs or small hairpin RNAs into siRNAs. siRNAs can also be exogenously (artificially) introduced into cells to bring about the specific knockdown of a gene of interest. Thus, any gene of which the sequence is known can in principle be targeted based on sequence complementarity with an appropriately tailored siRNA. The double-stranded RNA molecule or a metabolic processing product thereof is capable of mediating target-specific nucleic acid modifications, particularly RNA interference and/or DNA methylation. Preferably at least one RNA strand has a 5'- and/or 3'-overhang. Preferably, one end of the double-strand has a 3'-overhang from 1-5 nucleotides, more preferably from 1-3 nucleotides and most preferably 2 nucleotides. The other end may be blunt-ended or has up to 6 nucleotides 3'-overhang. In general any RNA molecule suitable to act as siRNA is envisioned in the present invention. The most efficient silencing was so far obtained with siRNA duplexes composed of 21-nt sense and 21-nt antisense strands, paired in a manner to have a 2-nt 3'- overhang. The sequence of the 2-nt 3' overhang makes a small contribution to the specificity of target recognition restricted to the unpaired nucleotide adjacent to the first base pair (Elbashir et al. 2001). 2'-deoxynucleotides in the 3' overhangs are as efficient as ribonucleotides, but are often cheaper to synthesize and probably more nuclease resistant.

A "shRNA" in accordance with the present invention is a short hairpin RNA, which is a sequence of RNA that makes a tight hairpin turn that can also be used to silence gene expression via RNA interference. shRNA utilizes the U6 promoter for its expression. The shRNA hairpin structure is cleaved by the cellular machinery into siRNA, which is then bound to the RNA-induced silencing complex (RISC). This complex binds to and cleaves mRNAs which match the siRNA that is bound to it.

siRNAs and shRNAs of the present invention are preferably chemically synthesized using appropriately protected ribonucleoside phosphoramidites and a conventional DNA/RNA synthesizer. Suppliers of RNA synthesis reagents include Proligo (Hamburg, Germany), Dharmacon Research (Lafayette, CO, USA), Pierce Chemical (part of Perbio Science, Rockford, IL , USA), Glen Research (Sterling, VA, USA), ChemGenes (Ashland, MA, USA), and Cruachem (Glasgow, UK).

The term "ribozymes" refers to ribonucleic acid enzyme, also called RNA enzyme or catalytic RNA, that are RNA molecules that are capable of catalysing chemical reactions. Many naturally occurring ribozymes catalyse either their own cleavage or the cleavage of other RNAs, but they have also been found to catalyse the aminotransferase activity of the ribosome. Examples of well-characterized small self-cleaving RNAs are the hammerhead, hairpin, hepatitis delta virus, and in vitro-selected lead-dependent ribozymes. The principle of catalytic self-cleavage has become well established in the last 10 years. The hammerhead ribozymes are characterized best among the RNA molecules with ribozyme activity. Since it was shown that hammerhead structures can be integrated into heterologous RNA sequences and that ribozyme activity can thereby be transferred to these molecules, it appears that catalytic antisense sequences for almost any target sequence can be created, provided the target sequence contains a potential matching cleavage site.

The basic principle of constructing hammerhead ribozymes is as follows: An interesting region of the RNA, which contains the GUC (or CUC) triplet, is selected. Two oligonucleotide strands, each with 6 to 8 nucleotides, are taken and the catalytic hammerhead sequence is inserted between them. Molecules of this type were synthesized for numerous target sequences. They showed catalytic activity in vitro and in some cases also in vivo. The best results are usually obtained with short ribozymes and target sequences.

The method of the invention using transposon-mediated mutagenesis in spermatogonial stem cells in combination with a transgene, gene trap transgenes, such as a polyA trap, enhancer trap, promoter trap or gene trap, siRNA, shRNA or ribozyme expression provides the advantage of having the possibility to verify copy number and, where appropriate, expression characteristics *in vitro* in a large number of candidate transgenic spermatogonial stem cells lines before producing the corresponding animal models. This is of particular interest as for these methods single copy introduction is advantageous as multiple insertions can make the identification of an underlying mutation causing a particular phenotype difficult. Subsequently, the spermatogonial stem cells obtainable or obtained by the method of the invention may be used in the convenient application of transgene expression, RNA interference or ribozyme cleavage applications *in vivo* in a variety of animal models, including animals such as rats, where similar approaches were so far not available.

In another preferred embodiment, the method of the invention further comprises the steps of identifying and/or cloning the mutated chromosomal site.

In accordance with the method of the invention it may be advantageous to determine the site of integration of the transposon prior to performing any further experiments, for example, generating non-human animals of the invention. Identification of the mutated site can be achieved by molecularbiological methods well-known to the person skilled in the art. For example, PCR-based technologies can be employed. A PCR is performed with one primer directed against the known sequence of the transposon and the resulting amplicons spanning the region surrounding the transposon are sequenced and mapped on the target genome preferably using basic bioinformatics methods, such as, for example, alignment via BLAST (an online bioinformatics tool available on the site maintained by the National Center for Biotechnology Information (NCBI) at http://www.ncbi.nlm.nih.gov/blast/Blast.cgi).
Further, it may be advantageous to clone the identified mutated chromosomal site in order to further examine the site of integration and the potential effects of the mutation. Methods for cloning are well-known to the person skilled in the art and are described for example in Sambrook et al., Molecular Cloning: A laboratory manual, third edition, Cold Spring Harbor Laboratory Press, 2000, which is herewith incorporated in its entirety.

The present invention further relates to a method for linking a phenotype with genetic information comprising the steps: a) producing a mutated spermatogonial stem cell by introducing at least one transposon into the cell; b) identifying the transposon insertion site; c) producing a non-human animal comprising the spermatogonial stem cells of a); and d) characterising the phenotype of the non-human animals of c); wherein phenotypical differences observed in comparison to animals not comprising the spermatogonial stem cells of a) are linked to the insertion site identified in b).

The term "phenotype" is well known to the skilled person and relates to any observable aspects of an organism, such as its morphology, development, or behaviour. In order to correlate a certain phenotype with genetic information, the so called *reverse genetic* approach is applied in the above described method of the invention. In a first step, mutagenic transposon "jumps" are induced in spermatogonial stem cells. For example, the Sleeping Beauty transposon possesses high transpositional activity in spermatogonial stem cells upon simple transfection. By combining gene trapping technology, antibiotic selection for gene trap events and high-throughput culturing techniques, a collection of several thousand mutant cell clones is generated, each having a mutagenic transposon insertion in a gene. Secondly, insertion sites are identified. Each transposon insertion is identified from the genome of mutagenised spermatogonial stem cells using, for example, PCR technologies or plasmid rescue out of genomic DNA. Insertion sites are then mapped on the genome of the corresponding animal using basic bioinformatic methods (BLAST). The identification of insertion sites as well as the mapping to genome data are well known to the skilled person. Next, mutant cell lines may be archived, e.g. by freezing for up to one year, and can be recovered back to culture, without any obvious morphological change. The production of viable offspring from mutagenised stem cells may be performed with thawed archived cells or directly after the cells were obtained. The spermatogonial cells are transplanted to repopulate testes of sterilized, wild-type recipient male animals. About 50-80% of the offspring originate from the transferred spermatogonial stem cells. The recipient males carrying transposon insertions in their sperm are bred with non-transgenic females to generate heterozygous and/or hemizygous offspring which are then crossbred to produce homozygous mutants for phenotyping. Phenotypic characterisation includes, for example, examination of mutants using a broad standardised phenotypic check-up. For example, the German Mouse Clinic (online resource to be found on the world wide web: www.mouseclinic.de) provides such a screening test with more than 240 parameters. Said screen is designated to the areas of behaviour, bone and cartilage development, neurology, clinical chemistry, eye development, immunology, allergy, steroidmetabolism, energy metabolism, lung function, vision and pain perception, molecular phenotyping, cardiovascular analyses and pathology.

In a preferred embodiment of the method of the invention said linking of a phenotype with genetic information is employed for the design of therapeutic and pharmaceutical intervention.

The data obtained from the phenotyping characterisation of animals comprising a particular mutated spermatogonial stem cell clone as described-above can then directly be correlated with the insertion site identified for this clone. Thus, a link between a certain genotype and its phenotypical expression can be established, providing useful information for therapeutic and pharmaceutical intervention.

in a preferred embodiment of the method for linking a phenotype with genetic information according to the present invention, the non-human animal of step c) is produced by introduction of spermatogonial stem cells having transposon introduction sites located within quantitative trait loci.

The term "quantitative trait loci" (QTLs) in accordance with the present invention are regions of DNA that are closely linked to the genes that underlie a particular phenotypic trait. QTLs are not necessarily genes themselves but can be used to map regions of the genome that contain genes involved in specifying a quantitative trait. Many years of phenotyping and detailed analysis of crosses of inbred animals, such as rats, have resulted in the initial localisation of hundreds of such loci involved in complex diseases and quantitative phenotypes. These quantitative trait loci demonstrate genetic linkage to many disease traits which are shared between humans and laboratory animals, such as rats, including hypertension, neuronal regeneration, ischemic cerebro- and cardiovascular disease and diabetes (Jacob, H.J. and A.E. Kwitek, 2002, "Rat genetics: attaching physiology and pharmacology to the Genome" Nat Rev Genet 3(1):33; Hubner, N. et al., 2005, "Integrated transcriptional profiling and linkage analysis for identification of genes underlying disease" Nat Genet 37(3):243).This approach of linking phenotypes with genetic information, which is also termed *forward genetic approach,* is in principle completely unbiased in the choice of target genes and resulted in a large number of genomic regions and potential candidate genes. This *forward genetic approach* needs to be combined with a *reverse genetic approach* that does not require the retroactive identification of the responsible gene.

One approach of creating mutants is to target and disrupt a gene of interest by homologous recombination; also referred to as *reverse genetics.* However, in spite of the growing knowledge about protein domains, protein-protein interactions and molecular structures, it is yet inadequate to reliably predict the biological process that will be affected by knocking out a particular gene. Another approach of obtaining mutant phenotypes is to induce loss-of-function mutations into genomes of model organisms in a random and genome-wide fashion, termed *forward genetics.* By using the non-human animals of the preferred embodiment of the method for linking a phenotype with genetic information, a forward genetic approach can be carried out, with the possibility for selecting mutations in genes or gene clusters of interest. Thus, this method provides a novel strategy for generating animal models, in particular rat models, with defined germline mutations in genes that regulate certain functions, such as cardiovascular function. The approach includes targeting the integration of transposable elements into known QTLs, e.g. known blood pressure QTLs at the level of spermatogonial stem cells *in vitro.* Mutant animals are then screened using a comprehensive panel of well-established physiological assays that are well known to the skilled person for the respective trait. Results from these assays will define novel genomic mutations directly linked to function under investigation.

In a more preferred embodiment of the method of linking a phenotype with genetic information, further insertions are introduced into a quantitative trait locus.

A quantitative trait locus may be further mutated using the "local hopping" property of transposons as described in the method of the invention infra. A transposon insertion site can be remobilized by the expression of the transposase recognizing the transposon elements responsible for mediating the excision either in spermatogonial stem cells in culture or at a later stage *in vivo.*

In a further preferred embodiment of the method of linking a phenotype with genetic information according to the present invention, the quantitative trait loci are associated with a disease selected from the group consisting of cardiovascular diseases, age-related diseases, cancer, obesity, diabetes mellitus or arthritis.

The term "cardiovascular diseases" refers to the class of diseases that involve the heart or blood vessels. Cardiovascular diseases indue, but are not restricted to, angina, atherosclerosis, cardiomyopathy, cerebrovascular disease, congenital heart disease, congestive heart failure, coronary artery disease, hypertrophic cardiomyopathy, myocardial infarction (heart attack) and high blood pressure (hypertension).

The term "age-related diseases" as used herein refers to diseases which can be characterized by the ioss of a cell's, organ's or organism's peak function that continues until its failure and death. In humans most physiological functions such as hearing, eyesight, taste, lung capacity, agility, immune response, adaptation to change etc. reach peak ability between the ages of 11 and 20 years old. After that age there is a slow decline in performance as the degenerative process of aging begins. Age-related diseases are well known in the literature, for example in Cutler & Mattson (Ageing Res. Rev. 2006, 5: 221).

In a preferred embodiment, the age-related disease is selected from the group consisting of fibrosis, inflammatory cardiomyopathy, heart hypertrophy, liver degeneration, skeletal muscle degeneration and chronic general inflammation.

Fibrosis generally relates to the formation of scar tissue in organs such as the liver or the heart. Inflammatory cardiomyopathy relates to an abnormal heart condition in which the heart is dilated (poor pumping power), restrictive (impaired ability of the heart to fill) or hypertrophic (enlarged heart). Heart hypertrophy is a disorder in which the heart muscle is so strong that it does not relax enough to fill the heart with blood and so has reduced pumping ability. Skeletal muscle degeneration generally refers to muscle wasting, sarcopenia as well as depletion of muscular tissues and fat stores. Chronic general inflammation generally relates to an infiltration of organs with inflammatory cells such as macrophages and neutrophile lymphocytes as well as increased levels of CRP and IL6. Emerging pathological evidence indicates that major chronic aging-related diseases such as atherosclerosis, arthritis, dementia, osteoporosis and cardiovascular diseases are inflammation-related.

Cancer, in accordance with the present invention refers to a class of diseases or disorders characterized by uncontrolled division of cells and the ability of these to spread, either by direct growth into adjacent tissue through invasion, or by implantation into distant sites by metastasis (where cancer cells are transported through the bloodstream or lymphatic system).

Obesity, in accordance with the present invention is a condition in which the natural energy reserve, stored in the fatty tissue of humans and other mammals, is increased to a point where it is associated with certain health conditions or increased mortality. Obesity is increasingly viewed as a serious and growing public health problem: excessive body weight has been shown to predispose to various diseases, particularly cardiovascular diseases, diabetes mellitus type 2, sleep apnea and osteoarthritis.

Diabetes mellitus, as used herein, is a syndrome characterized by disordered metabolism and inappropriately high blood sugar (hyperglycaemia) resulting from either low levels of the hormone insulin or from abnormal resistance to insulin's effects coupled with inadequate levels of insulin secretion to compensate. The three main forms of diabetes mellitus are: *type 1, type 2,* and *gestational diabetes* (occurring during pregnancy), which have similar signs, symptoms, and consequences, but different causes and population distributions. Type 1 diabetes is usually due to autoimmune destruction of the pancreatic beta cells. Type 2 diabetes is characterized by insulin resistance in target tissues, this causes a need for abnormally high amounts of insulin and diabetes develops when the beta cells cannot meet this demand. Gestational diabetes is similar to type 2 diabetes in that it involves insulin resistance as the hormones of pregnancy can cause insulin resistance in women genetically predisposed to developing this condition.

Arthritis, in accordance with the present invention refers to a group of conditions where there is damage caused to the joints of the body. There are many different forms of arthritis, each of which has a different cause. The most common form of arthritis, osteoarthritis (also known as degenerative joint disease) occurs following trauma to the joint, following an infection of the joint or simply as a result of aging. Other forms of arthritis are rheumatoid arthritis and psoriatic arthritis, which are autoimmune diseases in which the body is attacking itself. Septic arthritis is caused by joint infection. Gouty arthritis is caused by deposition of uric acid crystals in the joint that results in subsequent inflammation.

In another embodiment the invention relates to a transposon comprising a first set of transposase recognition sites specific for a first transposase flanking at least the following elements in 5' to 3' or 3' to 5' direction: i) a first selectable marker; ii) a second selectable marker comprising a second set of transposase recognition sites specific for a second transposase; wherein (a) said first set of transposase recognition sites are recognized by said first transposase but not by said second transposase; (b) said second set of transposase recognition sites are recognized by said second transposase but not by said first transposase; and (c) said second selectable marker is expressed upon removal of the second set of transposase recognition sites by the activity of the second transposase, resulting in expression of the second selectable marker.

Transposons and their method of action have been described hereinabove. Further, selectable markers have been described supra. Preferred are selectable markers which confer antibiotic resistance to cells expressing the marker, such as, for example, hygromycin or puromycin.
The transposon of the invention can be inserted into cells, preferably eukaryotic, more preferably vertebrate and most preferably spermatogonial cells. Upon successful integration, the second transposase can excise the second transposon comprising at least the second set of transposase recognition sequences and integrate said second transposon into another segment of the genome.

In a preferred embodiment of the transposon of the invention, the first set of transposase recognition sites have the nucleic acid sequence of SEQ ID NOs: 5 and 6 and the second transposase recognition sites have the nucleic acid sequence of SEQ ID NOs: 2 and 3.

In another preferred embodiment of the transposon of the invention, said transposon further comprises and/or the second transposase recognition sites of ii) flank elements selected from the group consisting of nucleotide sequences encoding a transgene or an siRNA, shRNA or a ribozyme.

Further elements which may be part of a transposon in accordance with the invention like transgenes, siRNA, shRNA or ribozymes have been described in detail above.

In a preferred embodiment of the method or the mutated spermatogonial stem cell of the invention, the transposon is a transposon of the invention.

The invention also relates to a method of generating a non-human animal having a mutation comprising the step of introducing a mutated spermatogonial stem cell of the invention into the non-human animal.

In connection with this embodiment of the invention, the mutation generated in the non-human animal is a mutation effected by the transposon insertion and optionally by subsequent transposon excision.

In a preferred embodiment of the method of generating a non-human animal, the method further comprises comprising the steps of: i) generating or obtaining a mutated spermatogonial stem cell of any one of claims 2 to 6 or 15; ii) transplanting the cells of step i) into the testes of sterilized, wild-type recipient male animals to repopulate said testes; iii) breeding the transgenic male animals obtained in step ii) with female animals to generate offspring; and iv) cross-breeding heterozygous or hemizygous offspring obtained in step iii) to generate homozygous non-human animals having a mutation.

The method of generating a non-human animal having a mutation comprises the step of transplanting the mutated spermatogonial stem cells of the invention to repopulate the testes of sterilized, wild-type recipient male animals subsequent to generating or obtaining said mutated spermatogonial cells. As a non-limiting example, about 0.5×10⁶ cultured mutated spermatogonial stem cells are transplanted when rats are used. Approximately 50-80% of the offspring originate from the transferred mutated spermatogonial stem cells. The recipient males carrying transposon insertions in their sperm are bred with wild-type females to generate heterozygous and/or hemizygous offspring which are then crossbred to produce homozygous mutants for phenotyping. Methods of transplanting spermatogonial stem cells into recipient male animals are well known to the person skilled in the art and are described, for example, in Hamra, K. et al., 2005, "Self renewal, expansion, and transfection of rat spermatogonial stem cells in culture", PNAS 102(48):17430.

The invention further relates to a non-human animal comprising the mutated spermatogonial stem cell of the invention.

In a preferred embodiment, the non-human animal of the invention is a vertebrate animal.

The term "vertebrate animals" in accordance with the present invention refers to animals which have a backbone or spinal cord. Vertebrates include mammals, amphibians, reptiles, birds and fish. The non-human animals may, for example, be mice, rats, hamsters, dogs, monkeys, rabbits, pigs, or cows.

In a more preferred embodiment, the vertebrate animal of the invention is a rodent.

The term "rodent" refers to an order of mammals that includes for example mice, rats, squirrels, chipmunks, gophers, porcupines, beavers, hamsters, gerbils, and guinea pigs.

In a particularly preferred embodiment the rodent animal of the invention is a rat.

The term "rat" refers to members of the genus *Rattus,* such as for example the black rat, *Rattus rattus,* and the brown rat, *Rattus norvegicus.* The laboratory rat is one of the most extensively studied model organisms for human disease and is the major animal model in the initial stages of drug development. In contrast to mice, a limitation of the rat model has been the lack of technology for generating "defined" genetic mutants. Such defined genetic mutants, where precise changes to a gene sequence or function are made without perturbing the rest of the genome, are critical for determining gene function with a high degree of certainty and for creating reliable genetic models for human disease. Due to the lack of technology for generating defined mutants in rats, genetic manipulation approaches were not successful in the rat genome so far and genome research in the rat to connect genetic backgrounds with certain phenotypes is lagging behind.

The method of the present invention, which is based on a transposon-based insertional mutagenesis technology in spermatogonial stem cells, now allows the production of novel rat models. As is shown in Example 1, a number of genes have been successfully tagged by Sleeping Beauty gene trap transposon insertion both *in vitro* as well as *in vivo.*

In another embodiment the invention relates to a method for saturating a genetic locus with mutations in a spermatogonial stem cell comprising the steps of: i) insertion of a transposon according to the invention into a genetic locus; ii) selecting for cells expressing the first selectable marker; iii) excising the second set of transposase recognition sites by the activity of the second transposase; and iv) selecting for cells expressing the second selectable marker.

Methods for insertion, selection and excision are well-known to the person skilled in the art and have in addition been described supra.
This method can be employed to mutate genes near a previously identified insertion site of a transposon of the invention in a 2 to 3 Mbp window. The transposon of the invention, the "carrier" transposon vector, comprises a second transposon, the "mutator" transposon. The carrier transposon is employed to distribute a large number of insertions in the genome. Once loci of special interest are identified, for example, quantitative trait loci, the mutator transposon can be remobilized for regional saturation mutagenesis of that genomic area. Remobilization can be effected, for example, by transfecting the mutated spermatogonial stem cells with a vector comprising an expressable transposase capable of effecting the excision of the mutator transposon from the carrier transposon and integration of the mutator transposon into the genome. Alternatively, the expressable gene for the transposase may be located within the mutator transposon under the control of an inducible promoter, such as, heat, chemical, or light sensitive promoters. For example, tetracycline inducible promoters or Dox-inducible promoters. Further suitable promoters are well-known to the person skilled in the art. Upon excision of the mutator transposon, a second selectable marker can be expressed allowing for selection of mutated spermatogonial cells with successful remobilization of the mutator transposon. Preferably, the transposase effecting excision and integration of the mutator transposon displays hyperactivity compared to wild-type transposases.
This method offers the advantage of giving the possibility to monitor the excision and subsequent reinsertion of a single copy of a transposon, preferably carrying a mutagenic gene trap, in the genome.
Thus, animals can be generated carrying a large number of mutations in chromosomal regions, where genes of interest are located in gene clusters.

Accordingly, in a preferred embodiment of the method of the invention the genetic locus is a quantitative trait locus.

This method is particularly advantageous to study genes in a quantitative trait locus and generally in genetic loci where genes are organized in gene clusters. It allows the phenotypic study of cells and/or animals which carry several mutations in their genome within a 2 or 3 Mbp window. Thus the contribution and interaction of genes may be studied in detail.

In another preferred embodiment of the method of the invention, steps iii) and iv) are effected in a non-human animal which is transgenic for the spermatogonial cells selected in step ii) of the method of the invention and has been generated according to the method of the invention.

As an alternative to carrying out all steps of the method of the invention, the step of remobilisation of the mutator transposon and the selection of clones where remobilization was successful can be carried out after animals have been generated which carry the carrier transposon in their genome, i.e. in vivo. The activation of the transposase specific for excision and integration of the mutator transposon may be effected as described supra. As an alternative method, the male recipient animals carrying the mutated spermatogonial cells may be bred to femal animals which are transgenic for the transposase excising and integrating the mutator transposon. The resulting offspring can be selected for heterozygous or hemizygous animals which are then cross-bred to generate homozygous animals.

In a further embodiment, the invention relates to a spermatogonial stem cell library comprising mutated spermatogonial stem cells of the invention.
A library in accordance with the invention comprises a large number of mutated spermatogonial stem cells which can be differentiated due to the specific mutations they are carrying. Advantageously, the spermatogonial stem cell clones of the library carry mutations which cover the entire genome. Further, the library may comprise mutated spermatogonial stem cells of different animal species. Preferably, the animals are rodents, such as mouse, rat or rabbit, but may also be selected from goat, sheep, cow, horse, or pig.
A stem cell library provides the basis for fast and reliable generation of animals transgenic for a specific site in the genome. In order to produce said animals the person skilled in the art would not necessarily have to carry out transposon-mediated mutagenesis and subsequently select and confirm successful mutation of the targeted site in the genome. Instead, he would merely have to repopulate testes and mate animals to obtain animals transgenic for a specific site in the genome.

Accordingly, in a preferred embodiment of the method of generating a non-human animal of the invention, the spermatogonial stem cell is obtained from a spermatogonial stem cell library of the invention.

### The figures show:

**Figure 1****.** Schematic overview over the transposon-mutagenesis method in spermatogonial stem cells. Gene trap events are selected for, identified and the mutated spermatogonial stem cells are transplanted into animals, which are subsequently used for breeding and, finally, phenotyping of homozygous mutant animals.
**Figure 2****.** Transposase activity can be increased up to 100x in Hela cells.
**Figure 3****.** High frequency Sleeping Beauty transposition in rat spermatogonial stem cells and detection of successful insertion based on the expression of the β-galactosidase enzyme.
**Figure 4****.** Examples of transposon-tagged alleles identified in rat spermatogonial stem cells. The chromosomal localisation, gene function, phenotype and QTL associated with are also indicated.
**Figure 5****.** Development of rat spermatogonia *in vivo* after genetic modification with Sleeping Beauty and clonal expansion of the cells in culture.
**Figure 6****.** Testes of recipient male animals can be colonised with the mutated cell lines selected for gene trap events. A) EGFP fluorescence indicating successful colonisation of the testis with the genetically modified cell line. B) Fully developed, elongated spermatids can be observed in the testis.
**Figure 7****.** "Local hopping": A chromosomal region around an initial transposon insertion site can be further mutated by re-stimulating the transposon mobilisation.
**Figure 8****.** Exemplary drawing of a vector which can be used for remobilization of a mutator transposon.

The invention will now be described by reference to the following examples which are merely illustrative and are not to be construed as a limitation of scope of the present invention.

### Example 1: Overview over the Transposon-based insertional mutagenesis.

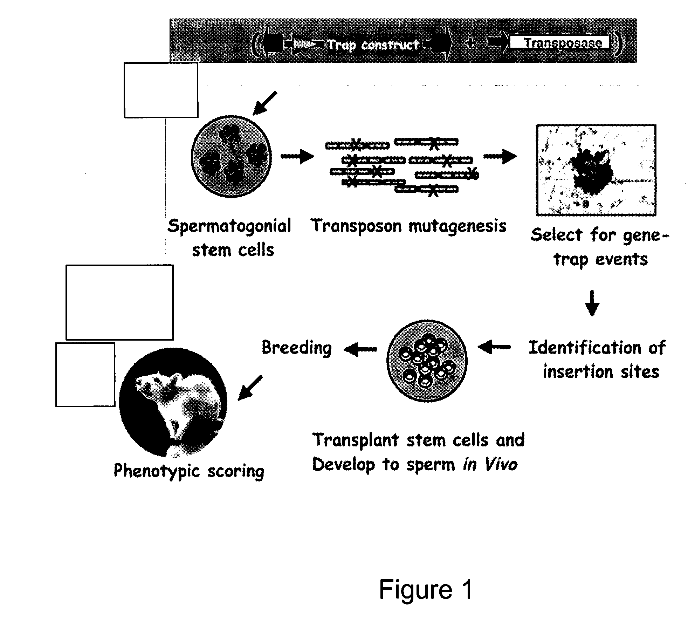

Cell culture of spermatogonial stem cells is carried out as described previously (e.g. Hamra, K. et al., 2005, "Self renewal, expansion, and transfection of rat spermatogonial stem cells in culture", PNAS 102(48):17430). Cultured spermatogonial stem cells are transfected with a transposon and, where necessary, with an additional construct encoding for the corresponding transposase enzyme. Gene trap events are selected for using antibiotic resistance and the insertion sites of selected clones are identififed using standard PCR protocols. Spermatogonial stem cells carrying an insertion at a site of interest are transplanted into the testes of sterilized, wild-type recipient male animals. The recipient males carrying transposon insertions in their sperm are bred with wild-type females to generate heterozygous and/or hemizygous offspring which are then crossbred to produce homozygous mutants for phenotyping. Figure 1 summarises the step outlined above.

### Example 2: The use of hyperactive transposases results in successful high efficiency integration

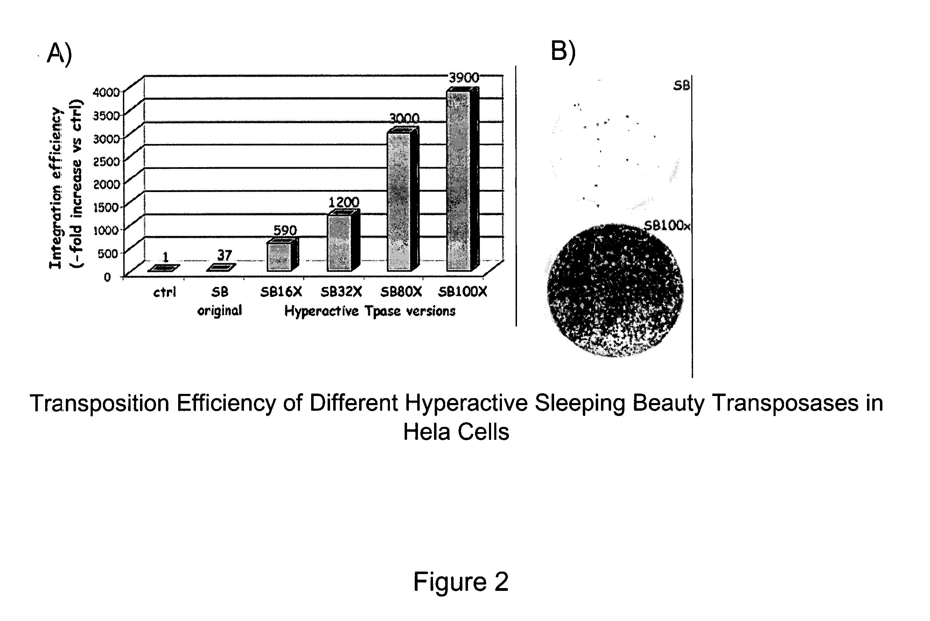

Transposition efficiency can be increased by employing hyperactive transposases as is shown in Figure 2. Using Hela cells, the transposition efficiency of the Sleeping Beauty transposase can be increased up to 100-fold as compared to normal Sleeping Beauty efficiency (transposition efficiency is expressed as the numbers of colonies with transposase divided by the number of colonies without transposase).

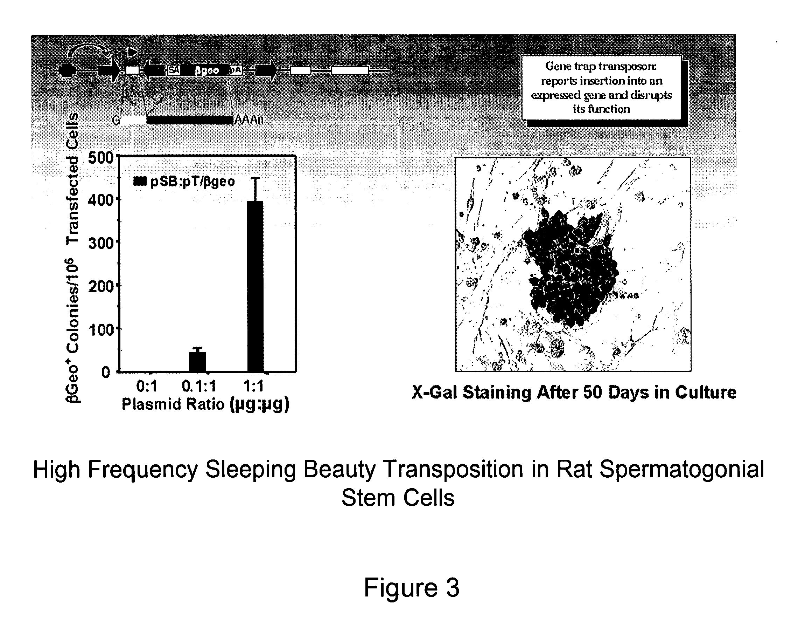

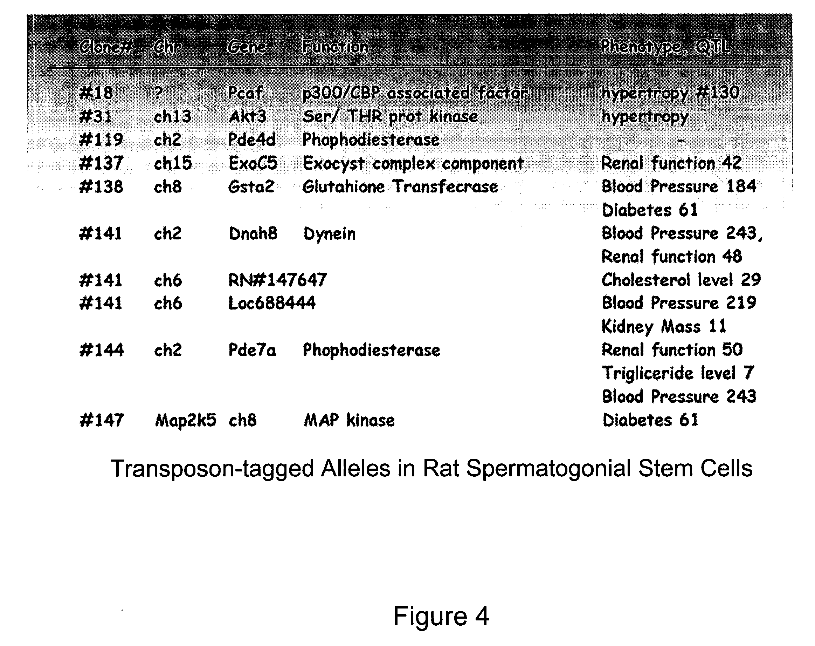

A high frequency Sleeping Beauty transposon was then transfected into rat spermatogonial stem cells at different ratios. Due to the presence of a β-geo transgene, successful integration can be monitored by X-gal staining as shown in Figure 3. Using the transposon-based insertional mutagenesis in rat spermatogonial stem cells a large number of different genes can be tagged by the transposon and subsequently identified. Figure 4 provides examples of genes successfully tagged as well as their chromosomal location, their phenotypic association as well as the QTLs they are associated with.

### Example 3: In vivo development of genetically modified rat spermatogonial stem cells

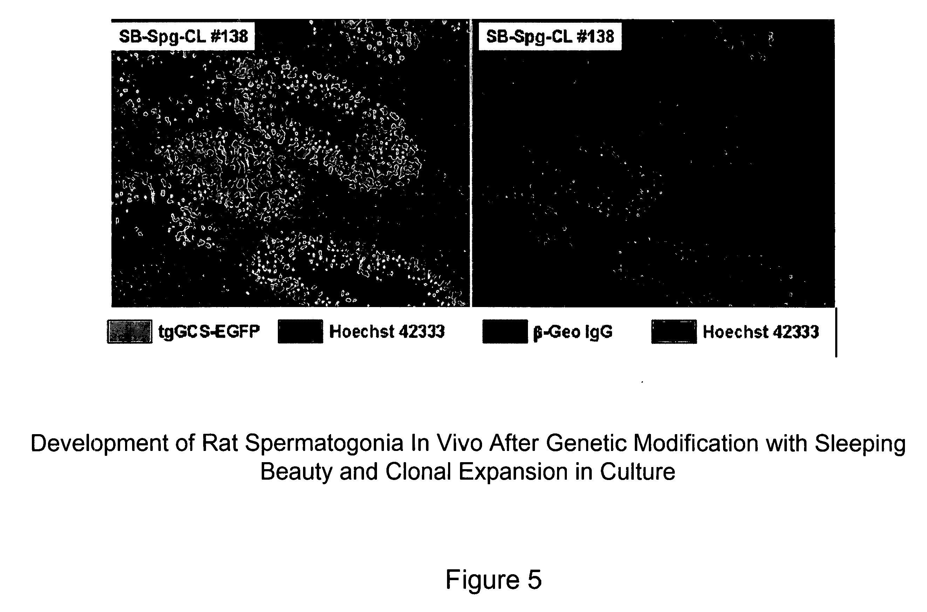

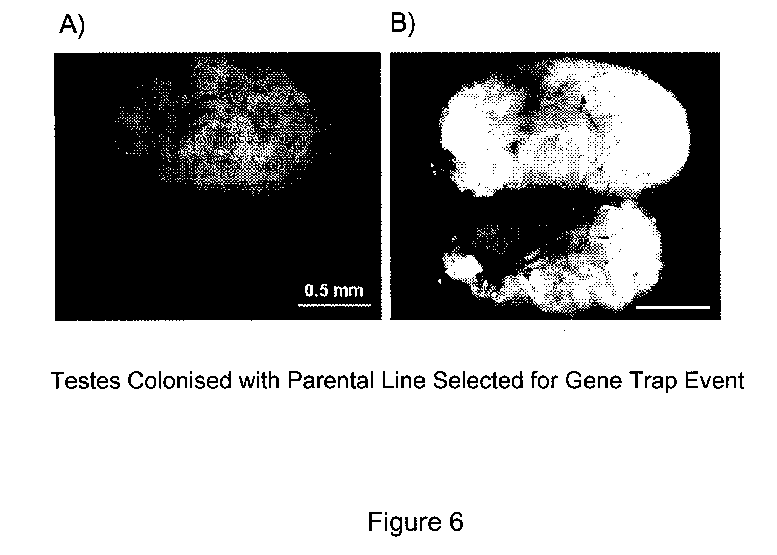

Rat spermatogonial stem cells modified with the Sleeping Beauty transposon and clonally expanded in culture develop normally *in vivo.* Figure 5 shows the successful *in vivo* development of rat spermatogonia, while Figure 6 shows that testes are colonised with the mutant cell line selected for a gene trap event. The integration of functional EGFP protein at the insertion site allows detection of the genetically modified rat spermatogonial stem cells *in vivo* (Figure 6A). The testes show fully developed elongated spermatids (Figure 6B).

### Example 4: Remobilisation of transposons in vivo and in vitro

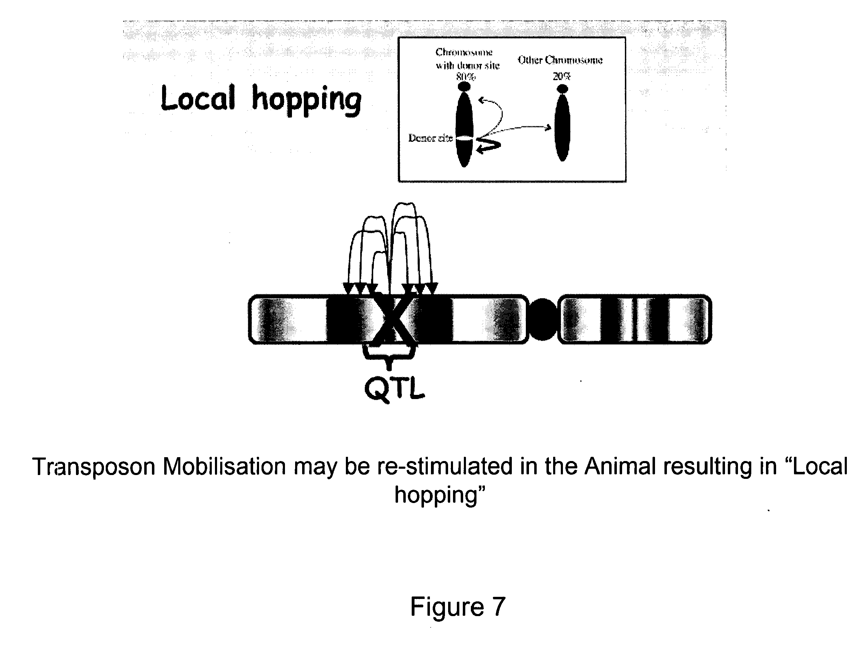

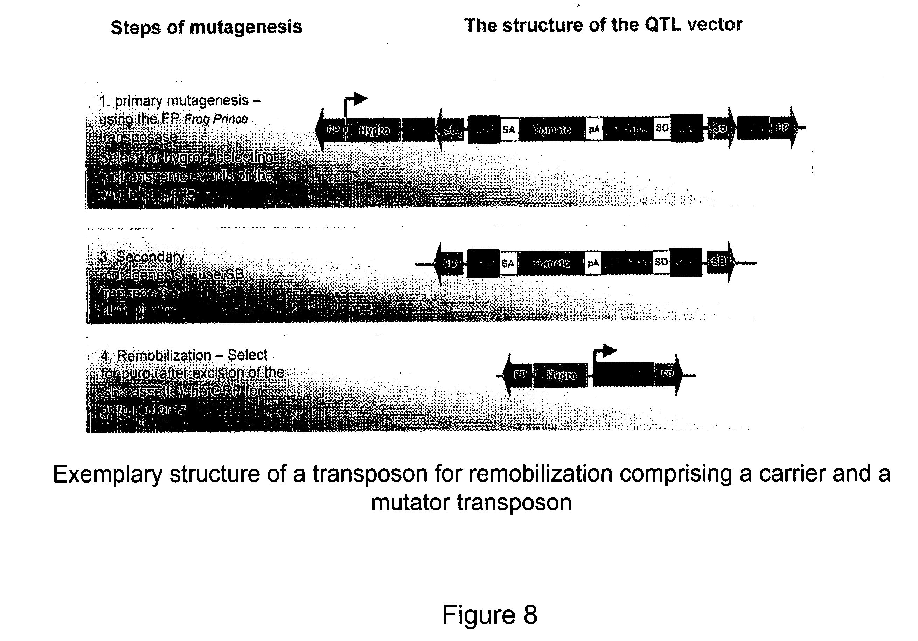

Transposon mobilisation may be re-stimulated in the animal, for example by using the Sleeping Beauty 100x high activity transposon. For example, a quantitative trait locus may be further mutated using the "local hopping" property of transposons. By remobilisation of the transposon, genes near a previously identified insertion site (in a 2 to 3 Mb window) can be mutated. Figures 7 and 8 exemplify said method.

### Example 5: Establishing new models of cardiovascular diseases.

To create new animal models to study the genetics of QTLs associated with cardiovascular diseases, for example in rat, 1,000 knock-out rat strains are generated carrying transposon gene insertions in target regions that contain either previously identified human disease genes or candidate genes suspected of playing a role in cardiovascular diseases. The selection is based on known or predicted biological function, genetic mapping studies in experimental animal models, data from published microarray studies and overlap with previously identified QTL regions. 50 mutant strains selected as high probability candidates for involvement in cardiovascular pathophysiology are subjected to detailed phenotyping assays to characterize any of several disease-related traits. The detailed phenotypic characterization of these 50 priority mutant strains will lead to the establishment of numerous novel models to better understand cardiovascular disease.

## Claims

1. A method for generating mutations in spermatogonial stem cells comprising introducing at least one transposon into the spermatogonial stem cells.

2. A mutated spermatogonial stem cell, obtainable by the method of claim 1.

3. The method of claim 1 or the mutated spermatogonial stem cell of claim 2,
wherein the at least one transposon is a eukaryotic transposon.

4. The method or the mutated spermatogonial stem cell of claim 3, wherein the transposon is the Sleeping Beauty transposon, the Frog Prince transposon, the piggyBac transposon or the Tol2 transposon.

5. The method or the mutated spermatogonial stem cell according to any one of claims 1 to 4, wherein the transposon comprises additional nucleotide sequences selected from the group consisting of nucleotide sequences encoding
a) a selectable marker;
b) a transgene; or
c) an siRNA, shRNA or a ribozyme.

6. The method of any one of claims 1 or 3 to 5 further comprising the steps of identifying and/or cloning the mutated chromosomal site.

7. A method for linking a phenotype with genetic information comprising the steps:
a) generating a mutated spermatogonial stem cell by introducing at least one transposon into the cell;
b) identifying the transposon insertion site;
c) generating a non-human animal comprising the spermatogonial stem cells of a); and
d) characterising the phenotype of the non-human animals of c);
wherein phenotypical differences observed in comparison to animals not comprising the spermatogonial stem cells of a) are linked to the insertion site identified in b).

8. The method of claim 7 wherein said linking of a phenotype with genetic information is employed for the design of therapeutic and pharmaceutical intervention.

9. The method of claim 7 or 8, wherein the non-human animal of step c) is produced by introduction of spermatogonial stem cells having transposon introduction sites located within quantitative trait loci.

10. The method of claim 9, wherein further insertions are introduced into a quantitative trait locus.

11. The method of claim 9 or 10, wherein the quantitative trait loci are associated with a disease selected from the group consisting of cardiovascular diseases, ageing-related diseases, cancer, obesity, diabetes mellitus or arthritis.

12. A transposon comprising a first set of transposase recognition sites specific for a first transposase flanking at least the following elements in 5' to 3' or 3' to 5' direction:
i) a first selectable marker;
ii) a second selectable marker comprising a second set of transposase recognition sites specific for a second transposase;
wherein
(a) said first set of transposase recognition sites are recognized by said first transposase but not by said second transposase;
(b) said second set of transposase recognition sites are recognized by said second transposase but not by said first transposase; and
(c) said second selectable marker is expressed upon removal of the second set of transposase recognition sites by the activity of the second transposase, resulting in expression of the second selectable marker.

13. The transposon of claim 12 wherein the first set of transposase recognition sites have the nucleic acid sequence of SEQ ID NOs:5 and 6 and the second transposase recognition sites have the nucleic acid sequence of SEQ ID NOs: 2 and 3.

14. The transposon of claims 12 or 13 wherein said transposon further comprises and/or the second transposase recognition sites of ii) flank elements selected from the group consisting of nucleotide sequences encoding a transgene or an siRNA, shRNA or a ribozyme.

15. The method or the mutated spermatogonial stem cell of any one of claims 1 to 3 wherein the transposon is a transposon according to any one of claims 12 to 14.

16. A method of generating a non-human animal having a mutation comprising the step of introducing the mutated spermatogonial stem cell of any one of claims 2 to 6 or 15 into the non-human animal.

17. The method of claim 16 further comprising the steps of:
i) generating or obtaining a mutated spermatogonial stem cell of any one of claims 2 to 6 or 15;
ii) transplanting the cells of step i) into the testes of sterilized, wild-type recipient male animals to repopulate said testes;
iii) breeding the transgenic male animals obtained in step ii) with female animals to generate offspring; and
iv) cross-breeding heterozygous or hemizygous offspring obtained in step iii) to generate homozygous non-human animals having a mutation.

18. A non-human animal comprising the mutated spermatogonial stem cell according to any one of claims 2 to 6 or 15.

19. The non-human animal of claim 18 which is a vertebrate animal.

20. The vertebrate animal of claim 19 which is a rodent.

21. The rodent animal of claim 20 which is a rat.

22. A method for saturating a genetic locus with mutations in a spermatogonial stem cell comprising the steps of:
i) insertion of a transposon according to any one of claims 12 to 14 into a genetic locus;
ii) selecting for cells expressing the first selectable marker
iii) excising the second set of transposase recognition sites by the activity of the second transposase; and
iv) selecting for cells expressing the second selectable marker.

23. The method of claim 22 wherein the genetic locus is a quantitative trait locus.

24. The method of claim 22 or 23 wherein steps iii) and iv) are effected in a non-human animal which is transgenic for the spermatogonial stem cells selected in step ii) of claim 22 and has been generated according to the method of claim 16 or 17.

25. A spermatogonial stem cell library comprising mutated spermatogonial stem cell of any one of claims 2 to 6 or 15.

26. The method of generating a non-human animal of claim 16 or 17, wherein the spermatogonial stem cell is obtained from a spermatogonial stem cell library of claim 25.
